# EUROPÄISCHE PATENTSCHRIFT

(11) **EP 1 973 590 B1**
(45) Veröffentlichungstag und Bekanntmachung des Hinweises auf die Patenterteilung: **18.05.2016**
(21) Anmeldenummer: 07700065.1
(22) Anmeldetag: 11.01.2007
(51) Int. Cl.: A61J 1/20, A61M 39/18, A61L 2/26, A61L 2/08, A61M 5/00

(54) **BEHÄLTER ZUR EINBRINGUNG ZUMINDEST EINES UNSTERILEN GEFÄSSES IN EINEN STERILEN BEREICH**
CONTAINER FOR PLACING AT LEAST ONE UNSTERILE VESSEL INTO A STERILE REGION
CONTENANT PERMETTANT D'AMENER AU MOINS UN RÉCIPIENT NON STÉRILE DANS UN ENVIRONNEMENT STÉRILE

(30) Priorität: 18.01.2006 AT 742006; 13.04.2006 AT 6432006
(43) Veröffentlichungstag der Anmeldung: 01.10.2008
(73) Patentinhaber: Pipelka, Friedrich, 3400 Klosterneuburg (AT)
(72) Erfinder: Pipelka, Friedrich, 3400 Klosterneuburg (AT)
(74) Vertreter: Sonn & Partner Patentanwälte
(86) Internationale Anmeldenummer: PCT/AT2007/000008
(87) Internationale Veröffentlichungsnummer: WO 2007/082325

(56) Entgegenhaltungen:
- EP-A- 1 287 804
- WO-A-03/039632
- DE-A1- 10 005 813
- US-A1- 2005 137 566
- US-B2- 6 568 434

## Beschreibung

Die Erfindung betrifft einen Behälter zur Aufnahme zumindest eines eine entnehmbare Substanz enthaltenden unsterilen Gefäßes und zur Einbringung des zumindest einen unsterilen Gefäßes in einen sterilen Bereich, mit zumindest zwei miteinander verbindbaren Teilen, welche in miteinander verbundenem Zustand zur Aufnahme des zumindest einen Gefäßes ausgebildet sind, wobei ein Behälterteil eine Einrichtung zur Entnahme der jeweiligen Substanz aus dem zumindest einen Gefäß aufweist.

Unter den Begriff Substanz können medizinische Flüssigkeiten, aber auch Trockensubstanzen, welche vor der Anwendung mit Hilfe eines flüssigen Lösungsmittels gemischt werden, fallen. Auch Substanzen, wie sie beispielsweise in der Industrie unter Reinraumbedingungen verwendet werden, sind denkbar. Die Erfindung ist insbesondere zur Aufnahme von Behältern mit einem durchstechbaren Gummistopfen, welche eine entsprechende Substanz enthalten, gerichtet.

Aufgrund des sensiblen Inhaltes solcher Gefäße lassen sich derartige Gefäße, insbesondere Stechampullen, nicht sterilisieren. Derartige unsterile Gefäße dürfen nicht in den sterilen Bereich, beispielsweise einen Operationssaal, eingebracht werden. Üblicherweise findet daher ein Übergang der im unsterilen Gefäß befindlichen Substanz in den sterilen Bereich mit Hilfe von Spritzen und Einstechkanülen und/oder durch Zuhilfenahme von speziellen Transfereinheiten statt. Dieser Vorgang ist weiter unten genauer beschrieben. Die Handhabung erfordert zumindest eine Person im sterilen und eine Person im unsterilen Bereich und ist daher besonders aufwändig und somit nachteilig.

Die EP 1 287 804 A2 betrifft eine Vorrichtung zum Zusammenführen von Komponenten unter sterilen Bedingungen, bei der zwei Behälter über einen Adapter mit zwei miteinander verbundenen Nadeln in Verbindung stehen und in einer Weichblisterverpackung versiegelt sind. Zur Mischung werden die Behälter zueinander bewegt und vor der Anwendung die Weichblisterverpackung geöffnet.

Die WO 03/039632 A2 beschreibt ein Gefäß für eine Substanz, beispielsweise ein Medikament, welches in ein Gehäuse in Form einer Spritze eingebracht werden kann und von einer Umhüllung umgeben und versiegelt ist.

Die DE 100 05 813 A1 beschreibt ein Mischbesteck zur Herstellung einer anwendungsfertigen Lösung eines in einem Behälter bereitgestellten Medikaments durch Mischung des Medikaments mit einem Lösungs- oder Verdünnungsmittel. Zu diesem Zweck ist ein Behälter in einem spritzenartigen Gefäß angeordnet und wird vor der Anwendung durch eine in der Spritze befindliche Hohlnadel geöffnet und mit der Lösung gemischt.

Eine Vorrichtung zur Handhabung eines Gefäßes mit einer flüssigen medizinischen Substanz der gegenständlichen Art ist aus der US 6 568 434 B2 bekannt. Dabei wird das Gefäß mit der medizinischen Substanz zwischen zwei Behälterteilen, welche miteinander verbunden werden, angeordnet. Ein Behälterteil weist eine Einrichtung zur Entnahme der Flüssigkeit aus dem Gefäß in Form einer Nadel auf. Die Handhabung des Behälters während der Übergabe vom unsterilen in den sterilen Bereich ist jedoch nach wie vor relativ aufwändig.

Die Aufgabe der vorliegenden Erfindung besteht daher in der Schaffung eines oben genannten Behälters, mit dem zumindest ein eine Substanz enthaltendes unsteriles Gefäß ohne Gefahr der Verunreinigung in einen sterilen Bereich eingebracht werden kann. Der Behälter soll möglichst einfach und kostengünstig aufgebaut sein, so dass eine wirtschaftliche Anwendung auch als Wegwerfprodukt möglich ist. Der Behälter soll weiters möglichst einfach handhabbar sein.

Gelöst wird die erfindungsgemäße Aufgabe durch einen oben genannten Behälter, bei dem die Behälterteile sterilisiert sind und von einer aus zumindest zwei Teilen bestehenden, abnehmbaren Umhüllung umgeben sind, wobei die Behälterteile durch jeweils zugeordnete Umhüllungsteile umgeben sind, so dass die Behälterteile beim Trennen der Behälterteile zum Öffnen des Behälters nicht berührt werden müssen, und wobei die Umhüllungsteilezum Entfernen der Umhüllung abziehbar sind. Erfindungsgemäß wird das zumindest eine unsterile Gefäß mit der jeweiligen Substanz in den Behälter aus zwei Teilen im unsterilen Bereich eingebracht.

Danach wird die Umhüllung abgenommen, ohne die Teile des Behälters zu berühren und nunmehr der Behälter ohne Umhüllung, welcher sterilisiert worden ist, in den sterilen Bereich eingebracht. Über die zumindest eine Einrichtung zur Entnahme der jeweiligen Substanz aus dem zumindest einen Gefäß, welche verschiedenartig ausgebildet sein kann, wird nunmehr die jeweilige Substanz des zumindest einen unsterilen Gefäßes im sterilen Bereich entnommen und anforderungsgemäß verwendet. Bei mehreren Gefäßen innerhalb des Behälters können die Substanzen auch gleichzeitig entnommen und während der Entnahme gemischt werden. Der erfindungsgemäße Behälter lässt sich relativ kostengünstig herstellen und darüber hinaus einfach anwenden.

Die aus zumindest zwei Teilen bestehende abnehmbare Umhüllung ist vorzugsweise zumindest teilweise aus elastischem Material, insbesondere elastischem Kunststoff, gebildet. Bei Verwendung eines solchen dehnbaren Materials kann die Umhüllung leicht über die Teile des Behälters angeordnet werden und ebenso leicht vor der Übergabe des Behälters in den sterilen Bereich von diesem abgenommen werden.

Um die Abnahme der Umhüllung zu erleichtern, kann an jedem Teil der Umhüllung ein Fortsatz vorgesehen sein, an welchem die Umhüllung gegriffen und abgezogen werden kann.

Gemäß einem weiteren Merkmal der Erfindung weisen die Umhüllungsteile Ringelemente auf, welche in geschlossenem Zustand des Behälters vorzugsweise einander berühren. Diese Ringelemente können aus härterem Material als die übrige Umhüllung hergestellt sein und erleichtern das Anordnen der Umhüllung über dem Behälter und das Entfernen derselben.

Um ein ungewolltes Entfernen der Umhüllungsteile vom Behälter zu verhindern, können die Umhüllungsteile Elemente zur lösbaren Verbindung mit den Behälterteilen aufweisen, welche durch komplementär gestaltete Elemente auf den Behälterteilen und den Umhüllungsteilen, beispielsweise Nuten und entsprechende Ausbuchtungen, gebildet sind. Bevor die Umhüllungsteile von den Behälterteilen abgenommen werden, müssen diese lösbaren Verbindungselemente geöffnet bzw. überwunden werden.

Ein weiterer Vorteil kann erzielt werden, wenn eine Einrichtung zur Lösung der Verbindungselemente bei vollständig geschlossenem Behälter vorgesehen ist, welche durch komplementär gestaltete, schräge Endflächen der Umhüllungsteile bzw. deren Ringelemente gebildet ist, so dass bei vollständig geschlossenem Behälter die Verbindungselemente der Umhüllungsteile mit den Behälterteilen lösbar sind. Dadurch wird erreicht, dass beim Verbinden der Behälterteile nach Aufnahme des zumindest einen Gefäßes die Verbindung zwischen den Umhüllungsteilen und den Behälterteilen automatisch gelöst wird und somit die Entfernung der Umhüllung einfacher möglich wird.

Beim Schließen der Behälterteile bewirken die aneinander anliegenden schrägen Endflächen der Umhüllungsteile bei gleichzeitiger Verschiebung eine radiale Bewegung derselben, durch welche ein Lösen der Verbindungselemente möglich wird.

Der Behälter kann aus zumindest zwei im Wesentlichen gleich großen Behälterteilen bestehen. Dadurch ist die Handhabung einfacher, da an beiden Teilen des Behälters genügend Platz für die Finger des manipulierenden Personals vorhanden ist. Dies bezieht sich auf die übliche Größe von Stechampullen, welche beispielsweise 20 mm Durchmesser und 50 mm Höhe aufweisen. Natürlich ist eine Anwendung für mehrere Gefäße und andere Gefäße in unterschiedlicher Größe oder Form ebenfalls denkbar.

Die Behälterteile können über eine Steckverbindung miteinander verbindbar sein. Eine derartige Steckverbindung kann besonders leicht hergestellt und auch einfach bedient werden.

Ebenso ist es möglich, die Behälterteile über ein Gewinde miteinander zu verbinden, wobei das Gewinde idealerweise für eine leichte Handhabung nur einen Bruchteil einer 360° Umdrehung, beispielsweise 90° oder 180°, aufweist.

Um eine ungewollte Trennung der miteinander verbundenen Behälterteile des Behälters zu verhindern, können Rastnasen oder dgl. vorgesehen sein, welche vor einem Trennen der Behälterteile überwunden werden müssen. Ebenso können zur Sicherstellung einer Erstöffnung der Teile des Behälters bestimmte Einrichtungen, welche beim Öffnen zerstört, beispielsweise aufgerissen, werden müssen, vorgesehen sein.

Insbesondere für die Anwendung bei nur einem Gefäß in Form einer zylindrischen Stechampulle weisen die Behälterteile bevorzugt zylindrische Form auf.

Um eine einfache, rasche und kostengünstige Herstellung zu erlauben, sind die Behälterteile und die Umhüllungsteile vorzugsweise aus Kunststoff gebildet.

Um das im Behälter angeordnete zumindest eine Gefäß bzw. dessen jeweiligen Inhalt beobachten zu können, sind die Behälterteile und Umhüllungsteile vorzugsweise aus transparentem Kunststoff gebildet. Dabei ist es natürlich auch möglich, nur einen Teil des Behälters aus transparentem Kunststoff herzustellen.

Um eine Sterilisation des Behälters zu ermöglichen, ist der Kunststoff vorzugsweise sterilisierbar, insbesondere gammasterilisierbar.

Dabei können die Teile des Behälters und Umhüllungsteile beispielsweise aus Polyolefinen, insbesondere Polypropylen oder Polyäthylen, gebildet sein. Grundsätzlich sind aber sämtliche Kunststoffe für die Herstellung des Behälters möglich.

Die Teile des Behälters und die Teile der Umhüllung werden gemäß einem weiteren Merkmal der Erfindung im Spritzgussverfahren hergestellt. Dieses Verfahren lässt bei entsprechend hoher Stückzahl eine besonders kostengünstige Herstellung zu. Dadurch kann das Produkt auch als Einwegprodukt verwendet werden.

Die an einem Teil des Behälters angeordnete zumindest eine Entnahmeeinrichtung kann durch eine Durchstechmembran gebildet sein, welche von der Nadel einer entsprechenden Spritze durchstochen werden muss, bevor die Öffnung des zumindest einen unsterilen Gefäßes, insbesondere der durchstechbare Gummistopfen einer Stechampulle, ebenfalls von der Nadel durchstochen wird.

Alternativ dazu kann die zumindest eine Entnahmeeinrichtung auch durch zumindest eine Nadel gebildet sein. Die zumindest eine Nadel kann bei entsprechender Manipulation durch den Gummistopfen der Stechampulle gedrückt werden, worauf die Substanz des Gefäßes entnommen werden kann. Wichtig dabei ist, dass die Entnahmeeinrichtung so ausgebildet ist, dass bei abgenommener Umhüllung keine Verunreinigung der im Gefäß befindlichen Substanz stattfinden kann, bevor der Behälter in den sterilen Bereich transferiert wird. Dies kann beispielsweise mittels einer in der Entnahmeeinrichtung befindlichen Verschlusskappe oder dgl. erfolgen.

Vorzugsweise ist die zumindest eine derartige Nadel aus Kunststoff gebildet. Bei Kunststoffnadeln ist das Verletzungsrisiko geringer und darüber hinaus lassen sich derartige Kunststoffnadeln leichter und billiger herstellen.

Alternativ dazu kann die zumindest eine Nadel jedoch auch aus Metall gebildet sein.

Das beispielsweise durch zumindest eine Nadel gebildete Element im Behälter kann mit zumindest einem Verbindungsstück verbunden sein, über welches zumindest ein Gerät zur Entnahme der Substanz aus dem Gefäß angeschlossen werden kann.

Ein derartiges Verbindungsstück kann beispielsweise durch einen Schnellverschluss, insbesondere einen genormten Schnellverschluss, wie z.B. einen so genannten Luer- bzw. Luer-Lock-Verschluss, der zur Verbindung von Kanülen, Spritzen und Infusionsschläuchen im medizinischen Bereich genormt ist, gebildet sein. Jede andere Form der Verbindungsmöglichkeit mit einer eventuell erforderlichen Applikationseinrichtung ist jedoch auch denkbar.

Vorteilhafterweise ist eine sterile Verpackung des Behälters, beispielsweise in Form einer Blister-Verpackung, vorgesehen. Diese Verpackung wird vor der Anwendung geöffnet, die Teile des Behälters getrennt, das unsterile Gefäß eingebracht, die Teile zusammengesetzt und die Umhüllung entfernt, ohne die Teile des Behälters zu berühren und schließlich wird der Behälter in den sterilen Bereich eingebracht.

Die vorliegende Erfindung wird anhand der beigefügten Zeichnungen näher erläutert. Darin zeigen:
- Fig. 1: eine schaubildliche Darstellung der bisherigen Methode der Einbringung einer Substanz eines unsterilen Gefäßes in einen sterilen Bereich;
- Fig. 2: ein Schnittbild einer Ausführungsform eines erfindungsgemäßen Behälters;
- Fig. 3a: bis 3d schematische Darstellungen des Behälters zur Veranschaulichung der Anwendung;
- Fig. 4: das Schnittbild einer weiteren Ausführungsform eines erfindungsgemäßen Behälters; die
- Fig. 5: bis 9 Schnittbilder einer weiteren Ausführungsform eines erfindungsgemäßen Behälters in verschiedenen Stadien während einer Anwendung; die
- Fig. 10: bis 13 die Details A bis D aus den Fig. 6 bis 9 in vergrößerter Darstellung;
- Fig. 14: ein Schnittbild einer weiteren Ausführungsform eines erfindungsgemäßen Behälters für zwei Gefäße; und
- Fig. 15: das Schnittbild für die Ausführungsform gemäß Fig. 14 entlang der Schnittlinie XV-XV.

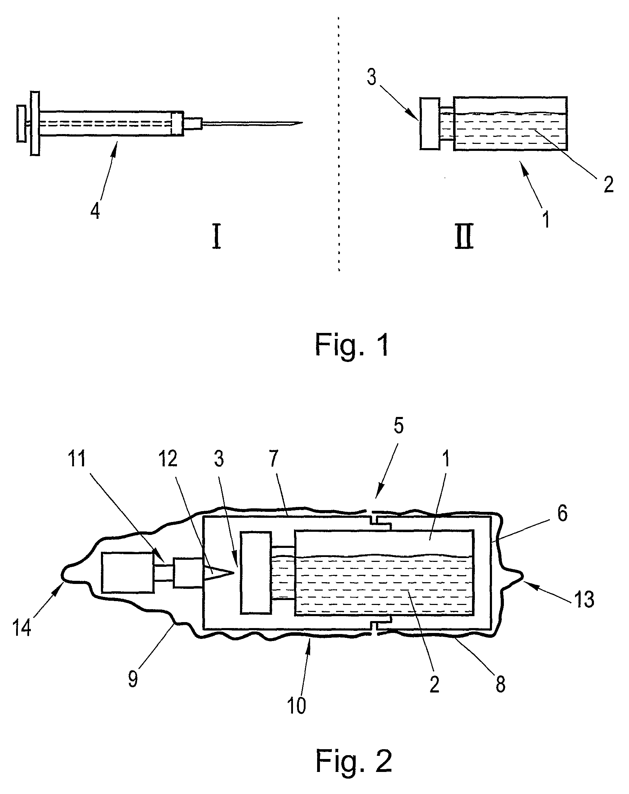

Fig. 1 zeigt eine schaubildliche Darstellung, wie derzeit eine Substanz 2, insbesondere Flüssigkeit, aus einem unsterilen Gefäß 1 aus einem unsterilen Bereich II in einen sterilen Bereich I eingebracht wird. Beim Gefäß 1 handelt es sich insbesondere um eine Stechampulle, die üblicherweise mit einem durchstechbaren Gummistopfen 3 zur Entnahme der Substanz 2 versehen ist. Da es sich bei der Substanz 2 üblicherweise um ein sensibles Medium handelt, eignet sich der Behälter 1 mit der darin enthaltenen Substanz 2 nicht zum Sterilisieren. Aus diesem Grund wird üblicherweise die Substanz 2 des Behälters 1 so in den sterilen Bereich I eingebracht, indem an der Grenze zwischen sterilem Bereich I und unsterilem Bereich II die Übertragung der Substanz 2 des Gefäßes 1, beispielsweise in eine Spritze 4, stattfindet. Zu diesem Zweck wird der Gummistopfen 3 des Gefäßes 1 mit einem Alkoholtupfer desinfiziert und danach mit der Nadel der Spritze 4 durchstochen und schließlich die Substanz 2 in die Spritze 4 gezogen. Danach wird die Spritze 4 mit der Substanz 2 im sterilen Bereich I beispielsweise zum Patienten befördert und die Substanz 2 appliziert. Dieser Vorgang ist relativ aufwändig und erfordert sowohl im unsterilen Bereich II, als auch im sterilen Bereich I entsprechendes Personal, welches zeitlich und örtlich zusammenspielen muss. Bei dem hier beschriebenen System ist diese zeitliche und örtliche Zusammenarbeit von zumindest zwei Personen nicht mehr erforderlich. Anstelle der Nadel an der Spritze 4 kann auch ein Schnellverschluss, beispielsweise ein Luer-LockVerschluss, angeordnet sein.

Fig. 2 zeigt eine Ausführungsform des erfindungsgemäßen Behälters 5 in geschnittener Darstellung. Der Behälter 5 besteht aus zumindest zwei Teilen 6, 7, welche miteinander verbindbar sind und im miteinander verbundenen Zustand zur Aufnahme des Gefäßes 1 ausgebildet sind. Im Falle einer Stechampulle, welche üblicherweise zylindrisch ausgebildet ist, ist der Behälter 5 ebenfalls vorzugsweise zylindrisch ausgeführt. Zusätzlich sind die Behälterteile 6, 7 von einer aus zumindest zwei Teilen 8, 9 bestehenden abnehmbaren Umhüllung 10 umgeben. Um eine Entnahme der Substanz 2 aus dem Gefäß 1 zu ermöglichen, wenn dieses Gefäß 1 im Behälter 5 angeordnet ist, muss weiters an einem Teil 7 des Behälters 5 eine Einrichtung 11 zur Entnahme der Substanz 2 aus dem Gefäß 1 vorgesehen sein. Diese Entnahmeeinrichtung 11 kann beispielsweise eine Nadel 12 umfassen, welche den Gummistopfen 3 des Gefäßes 1 durchsticht. Die aus zumindest zwei Teilen 8, 9 bestehende Umhüllung 10 dient dazu, dass der Behälter 5 zur Aufnahme des Gefäßes 1 geöffnet werden kann, ohne dass die Behälterteile 6, 7 berührt werden müssen und somit verunreinigt werden könnten. Nach der Aufnahme des Gefäßes 1 im Behälter 5 werden die Umhüllungsteile 8, 9 entfernt, ohne dass die Behälterteile 6, 7 berührt werden und der sterile Behälter 5 mit dem unsterilen Gefäß 1 in den sterilen Bereich I eingebracht wird. Zur leichteren Entfernung der Umhüllungsteile 8, 9 können entsprechende Fortsätze 13, 14 vorgesehen sein. Weiters ist es möglich, die freien Enden der Umhüllungsteile 8, 9 trompetenartig aufzuweiten (nicht dargestellt), woraus eine einfachere Handhabung resultiert. Nach dem Einsetzen des Gefäßes 1 und Verbinden der Behälterteile 6, 7 des Behälters 5 stoßen diese aufgeweiteten Enden der Umhüllungsteile 8, 9 aneinander und schützen somit den Behälter 5 wirkungsvoll vor Verschmutzung.

Die Behälterteile 6, 7 und auch die Umhüllungsteile 8, 9 sind vorzugsweise aus Kunststoff gebildet, der sterilisierbar, insbesondere gammasterilisierbar, sein soll. Um den Inhalt des Behälters 5 beobachten zu können, eignet sich transparenter Kunststoff besonders. Beispielsweise können die Behälterteile 6, 7 und auch die Umhüllungsteile 8, 9 aus Polyolefinen, insbesondere Polypropylen oder Polyäthylen, gebildet sein. Die Umhüllung 10 kann zumindest teilweise aus elastischem Material, insbesondere elastischem Kunststoff, gebildet sein.

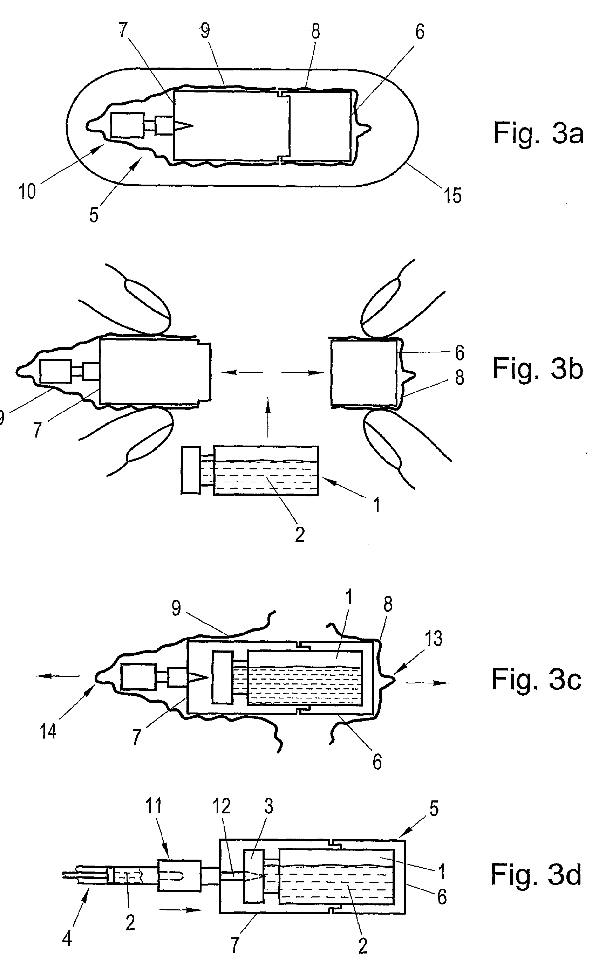

Die Anwendung des erfindungsgemäßen Behälters 5 wird anhand der Fig. 3a bis 3d näher erläutert. Gemäß Fig. 3a wird der Behälter 5 bestehend aus den Teilen 6 und 7 samt der aus den Teilen 8 und 9 bestehenden Umhüllung 10 in einer sterilen Verpackung 15 aufbewahrt. Vor der Anwendung wird die sterile Verpackung 15 geöffnet und der Behälter 5 durch Trennen der Behälterteile 6 und 7 geöffnet. Da die Behälterteile 6, 7 durch die Umhüllungsteile 8, 9 umgeben sind, müssen die Behälterteile 6, 7 nicht berührt werden. Gemäß Fig. 3b wird das die Substanz 2 enthaltende Gefäß 1 in den Behälter 5 eingebracht und die Behälterteile 6, 7 wieder miteinander verbunden. Danach wird gemäß Fig. 3c die Umhüllung 10 durch Abziehen der Umhüllungsteile 8, 9 entfernt. Zur Erleichterung der Entfernung kann die Umhüllung 10 an den Fortsätzen 13, 14 abgezogen werden. Der nunmehr sterile Behälter 5 mit dem unsterilen Gefäß 1 wird sodann in den sterilen Bereich eingebracht und kann mit sterilen Händen angefasst werden. Um die Substanz 2 des Gefäßes 1 entnehmen zu können, wird schließlich eine beispielsweise durch eine Nadel 12 gebildete Einrichtung 11 zur Entnahme der Substanz 2 aus dem Gefäß 1 durch den Gummistopfen 3 im Gefäß 1 gestochen (Fig. 3d). Danach kann mit einer entsprechenden Spritze 4 die Substanz 2 aus dem Gefäß 1 entnommen werden. Das Einstechen der Nadel 12 kann durch Verschiebung der Entnahmeeinrichtung 11 erfolgen. Die Entnahmeeinrichtung 11 beinhaltet auch eine Verschlusskappe oder dgl., welche verhindern soll, dass nach Abnahme der Teile 8, 9 der Umhüllung 10 die Substanz 2 im Gefäß 1 verunreinigt werden kann.

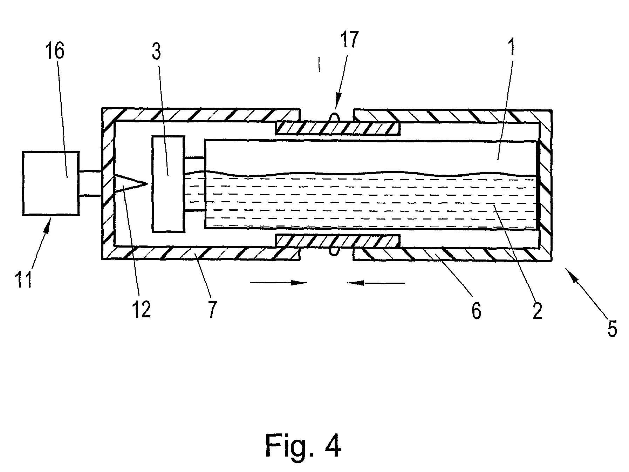

Fig. 4 zeigt eine alternative Ausführungsform der Erfindung, wobei der Behälter 5 aus zwei über eine Steckverbindung miteinander verbundenen Teilen 6, 7 besteht. An einem Behälterteil 7 ist eine Nadel 12 befestigt, welche mit einem Verbindungsstück 16, beispielsweise einem Schnellverschluss, wie einen Luer-Lock-Verschluss, in Verbindung steht. Die Nadel 12 wird durch eine Verschiebung der Behälterteile 6, 7 in axialer Richtung in den Gummistopfen 3 des Gefäßes 1 automatisch eingeschoben. Rastnasen 17 an der Verbindung zwischen den Behälterteilen 6 und 7 können eine ungewollte Öffnung des Behälters 5 verhindern. Durch die Rastnasen 17 wird verhindert, dass die sterile Umgebung durch den unsterilen Innenraum des Behälters 5 negativ beeinflusst wird.

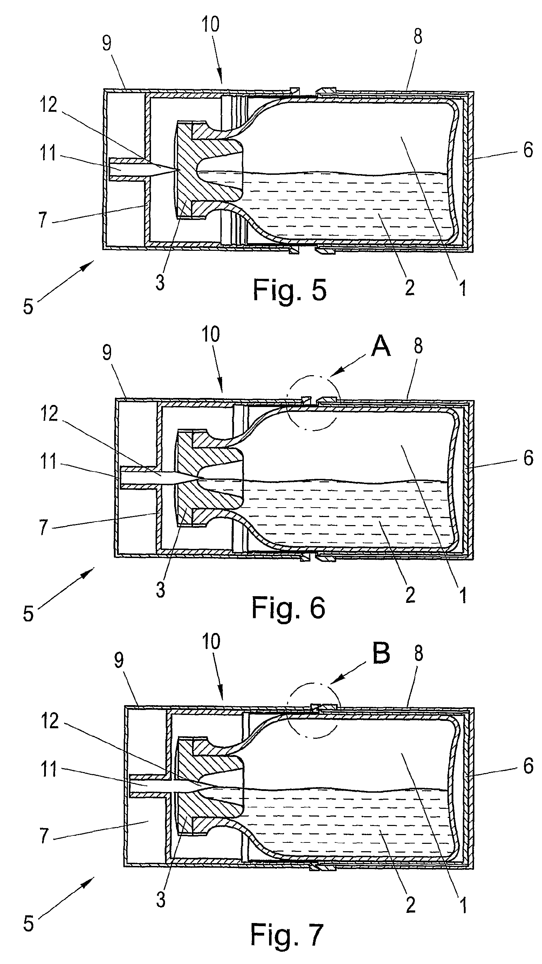

Die Fig. 5 bis 9 zeigen eine weitere Ausführungsform eines erfindungsgemäßen Behälters 5 in geschnittener Darstellung. Im Gegensatz zum Behälter 5 gemäß den Fig. 2 und 3a bis 3d besteht die Umhüllung 10 nicht aus Teilen 8, 9 aus elastischem Material, sondern aus festem Material. Nach dem Einbringen des unsterilen Gefäßes 1 mit der Substanz 2 in den Behälter 5 werden die beiden Behälterteile 6, 7 zueinander verschoben, wie in Fig. 5 dargestellt. Die Einrichtung 11 zur Entnahme der Substanz 2 aus dem Gefäß 1, welche im dargestellten Fall durch eine Nadel 12 gebildet ist, berührt gerade den Gummistopfen 3 des Gefäßes 1.

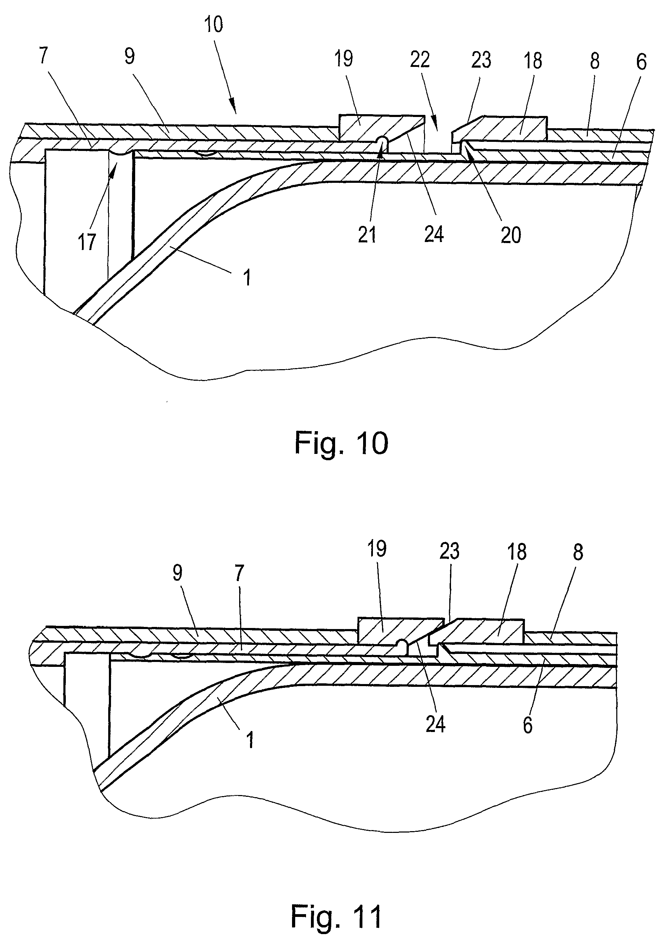

Die Fig. 6 bis 9 zeigen die verschiedenen Stufen bis zum vollständigen Zusammenschieben der Behälterteile 6, 7, wo es schließlich zu einer Entriegelung der Umhüllung 10 vom Behälter 5 kommt, so dass die Umhüllungsteile 8, 9 leichter vom Behälter 5 abgenommen werden können, bevor die Gesamtanordnung in den sterilen Bereich eingebracht wird. Hierzu wird auf die Fig. 10 bis 13 verwiesen, welche die Details A, B, C und D der Fig. 6, 7, 8 und 9 im Bereich der Verbindung zwischen den beiden Behälterteilen 6, 7 und Umhüllungsteilen 8, 9 in vergrößerter Darstellung zeigen.

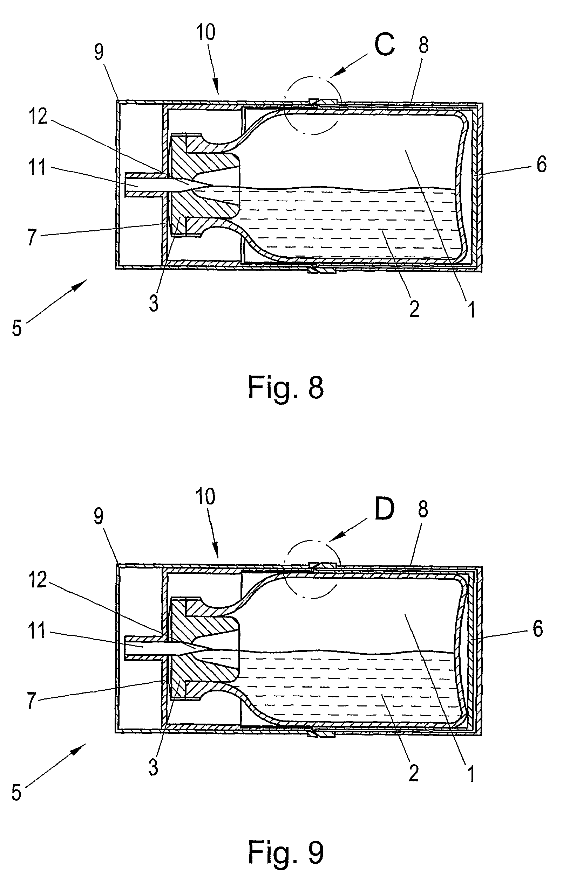

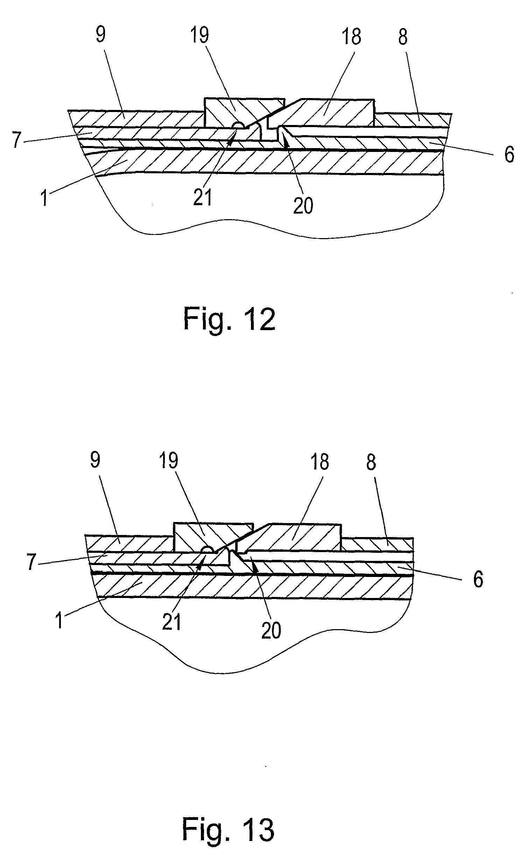

Entsprechend Detail A gemäß Fig. 10 besteht der Behälter 5 aus den beiden Behälterteilen 6, 7, welche über eine Rastnase 17 miteinander verriegelbar sind, so dass ein ungewolltes Öffnen des Behälters verhindert wird (siehe auch Fig. 4). Die Umhüllung 10 besteht aus zylindrischen Teilen 8, 9, welche Ringelemente 18, 19, vorzugsweise aus festerem Material als die übrigen Teile 8, 9, aufweisen. Die Ringelemente 18, 19 der Umhüllungsteile 8, 9 und die entsprechenden Behälterteile 6, 7 weisen erfindungsgemäß Verbindungselemente 20, 21 auf, welche beispielsweise durch komplementär gestaltete Elemente auf den Behälterteilen 6, 7 und den Ringelementen 18, 19 der Umhüllungsteile 8, 9 gebildet sein können. Diese Elemente 20, 21 dienen zur lösbaren Verbindung der Umhüllungsteile 8, 9 mit den Behälterteilen 6, 7, so dass ein ungewolltes Entfernen der Umhüllungsteile 8, 9 von den sterilen Behälterteilen 6, 7 verhindert wird. Die Ringelemente 18, 19 sind zur Bildung einer Einrichtung 22 zur Lösung der Verbindungselemente 20, 21 entsprechend gestaltet. Diese Einrichtung 22 zur Lösung der Verbindungselemente 20, 21 ist beispielsweise durch komplementär gestaltete schräge Endflächen 23, 24 der Ringelemente 18, 19 der Umhüllungsteile 6, 7 realisiert. Während des Zueinanderschiebens der Behälterteile 6, 7 mit den daran angeordneten Umhüllungsteilen 8, 9 kommt es zu einem Öffnen der Verbindungselemente 20, 21, über welche die Umhüllungsteile 8, 9 mit den Behälterteilen 6, 7 verbunden sind. Bei der Stellung gemäß Fig. 11 kommen die schrägen Endflächen 23, 24 der Ringelemente 18, 19 in Berührung. Bei weiterer Bewegung der Behälterteile 6, 7 zueinander kommt es entsprechend Fig. 12 zu einer Öffnung des Verbindungselements 21 des Umhüllungsteils 9 mit dem Behälterteil 7 und in weiterer Folge entsprechend Fig. 13 auch zu einer Lösung des Verbindungselements 20 zwischen Umhüllungsteil 8 und Behälterteil 6. In der Stellung gemäß Fig. 13 bzw. Fig. 9 sind die Behälterteile 6, 7 vollständig zueinander gepresst, und die durch die Rastnase 17 gebildete Verriegelung zwischen den beiden Teilen 6, 7 des Behälters 5 ist eingerastet. Die Verbindungselemente 20, 21 zwischen den Umhüllungsteilen 8, 9 und den Behälterteilen 6, 7 sind gelöst, wodurch die Umhüllungsteile 8, 9 besonders leicht und einfach abgeschoben werden können, bevor der Behälter 5 in den sterilen Bereich eingebracht wird. Die entsprechenden Verbindungselemente 20, 21 und die Einrichtung 22 zur Lösung derselben können natürlich auch direkt an den Umhüllungsteilen 8, 9 und nicht an den Ringelementen 18, 19 angeordnet sein.

Der Behälter 5 kann je nach Form, Größe und Anzahl des bzw. der aufzunehmenden Gefäße(s) 1 verschiedenartig ausgebildet sein. Ebenso kann die Einrichtung 11 zur Entnahme der Substanz 2 aus dem Gefäß 1 unterschiedlich gestaltet sein.

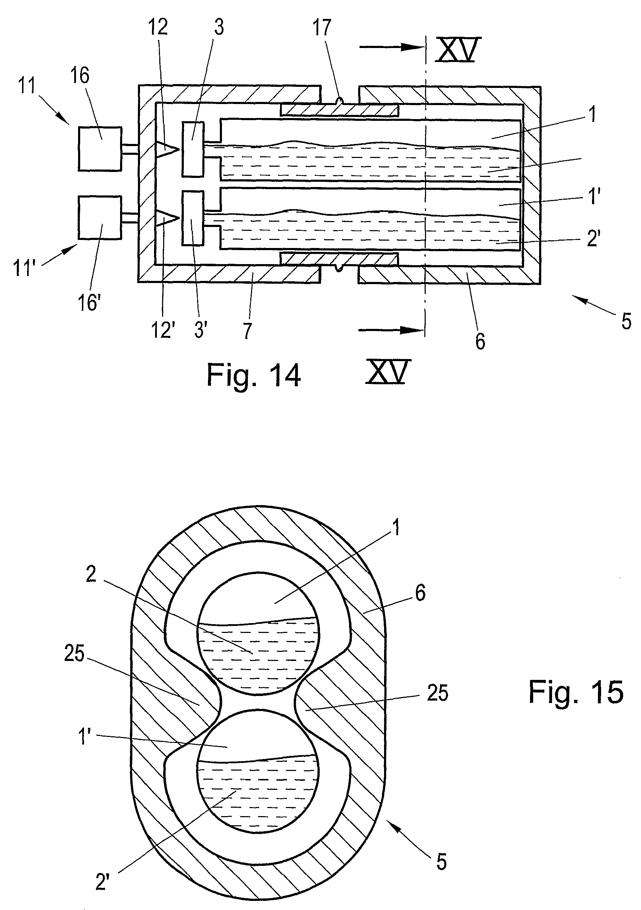

Fig. 14 zeigt eine alternative Ausführungsform der Erfindung, wobei der Behälter 5 für die Aufnahme zweier Gefäße 1, 1' mit darin enthaltenen Substanzen 2, 2' ausgebildet ist. Wie aus Fig. 15, welche das Schnittbild durch den Behälter 5 entlang der Schnittlinie XV-XV aus Fig. 14 zeigt, ersichtlich ist, kann der Behälter 5 bzw. dessen Teile 6, 7 im Wesentlichen oval ausgebildet sein und entsprechende Aufnahmen für die Gefäße 1, 1' aufweisen. Um die Gefäße 1, 1' im Wesentlichen in ihrer Lage zu halten, können Ausbuchtungen 25 im Teil 6 und bzw. oder Teil 7 des Behälters 5 angeordnet sein. Entsprechend der Anzahl der Gefäße 1, 1' sind mehrere Einrichtungen 11, 11' zur Entnahme der Substanzen 2, 2' aus den Gefäßen 1, 1' vorgesehen. Diese Entnahmeeinrichtungen 11, 11' sind in ähnlicher Weise, wie bei der Ausführungsform gemäß Fig. 4 durch zumindest eine Nadel 12, 12' und jeweils ein Verbindungsstück 16, 16' gebildet. Über die Verbindungstücke 16, 16' können die Substanzen 2, 2' aus den Gefäßen 1, 1' einzeln oder auch gleichzeitig entnommen werden. Natürlich können auch drei oder mehr Gefäße 1 innerhalb eines Behälters 5 angeordnet werden.

## Patentansprüche

1. Behälter (5) zur Aufnahme zumindest eines eine entnehmbare Substanz (2, 2') enthaltenden unsterilen Gefäßes (1, 1'), insbesondere einer Stechampulle mit einem durchstechbaren Gummistopfen (3), und zur Einbringung des zumindest einen unsterilen Gefäßes (1, 1') in einen sterilen Bereich (I), mit zumindest zwei miteinander verbindbaren Teilen (6, 7), welche in miteinander verbundenem Zustand zur Aufnahme des zumindest einen Gefäßes (1, 1') ausgebildet sind, wobei ein Behälterteil (7) zumindest eine Einrichtung (11, 11') zur Entnahme der jeweiligen Substanz (2, 2') aus dem zumindest einen Gefäß (1, 1') aufweist, **dadurch gekennzeichnet, dass** die Behälterteile (6, 7) sterilisiert sind und von einer aus zumindest zwei Teilen (8, 9) bestehenden, abnehmbaren Umhüllung (10) umgeben sind, wobei die Behälterteile (6, 7) durch jeweils zugeordnete Umhüllungsteile (8, 9) umgeben sind, so dass die Behälterteile (6, 7) beim Trennen der Behälterteile (6, 7) zum Öffnen des Behälters (5) nicht berührt werden müssen, und wobei die Umhüllungsteile (8, 9) zum Entfernen der Umhüllung (10) abziehbar sind.

2. Behälter (5) nach Anspruch 1, **dadurch gekennzeichnet, dass** die Umhüllung (10) zumindest teilweise aus elastischem Material, insbesondere elastischem Kunststoff, gebildet ist.

3. Behälter (5) nach Anspruch 1 oder 2, **dadurch gekennzeichnet, dass** an jedem Umhüllungsteil (8, 9) ein Fortsatz (13, 14) vorgesehen ist.

4. Behälter (5) nach einem der Ansprüche 1 bis 3, **dadurch gekennzeichnet, dass** die Umhüllungsteile (8, 9) Ringelemente (18, 19) aufweisen, welche in geschlossenem Zustand des Behälters (5) vorzugsweise einander berühren.

5. Behälter (5) nach einem der Ansprüche 1 bis 4, **dadurch gekennzeichnet, dass** die Umhüllungsteile (8, 9) Elemente (20, 21) zur lösbaren Verbindung mit den Behälterteilen (6, 7) aufweisen, welche durch komplementär gestaltete Elemente auf den Behälterteilen (6, 7) und den Umhüllungsteilen (8, 9) gebildet sind.

6. Behälter (5) nach Anspruch 5, **dadurch gekennzeichnet, dass** eine Einrichtung (22) zur Lösung der Verbindungselemente (20, 21) bei vollständig geschlossenem Behälter (5) vorgesehen ist, welche durch komplementär gestaltete schräge Endflächen (23, 24) der Umhüllungsteile (6, 7) bzw. deren Ringelemente (18, 19) gebildet ist, so dass bei vollständig geschlossenem Behälter (5) die Verbindungselemente (20, 21) der Umhüllungsteile (6, 7) mit den Behälterteilen (8, 9) lösbar sind.

7. Behälter (5) nach einem der Ansprüche 1 bis 6, **dadurch gekennzeichnet, dass** zumindest zwei im Wesentlichen gleich große Behälterteile (6, 7) vorgesehen sind.

8. Behälter (5) nach einem der Ansprüche 1 bis 7, **dadurch gekennzeichnet, dass** die Behälterteile (6, 7) über eine Steckverbindung miteinander verbindbar sind.

9. Behälter (5) nach einem der Ansprüche 1 bis 7, **dadurch gekennzeichnet, dass** die Behälterteile (6, 7) über ein Gewinde miteinander verbindbar sind.

10. Behälter (5) nach einem der Ansprüche 1 bis 9, **dadurch gekennzeichnet, dass** Rastnasen oder dgl. zum Verhindern einer ungewollten Trennung der miteinander verbundenen Behälterteile (6, 7) vorgesehen sind.

11. Behälter (5) nach einem der Ansprüche 1 bis 10, **dadurch gekennzeichnet, dass** die Behälterteile (6, 7) zylindrische Form aufweisen.

12. Behälter (5) nach einem der Ansprüche 1 bis 11, **dadurch gekennzeichnet, dass** die Behälterteile (6, 7) und die Umhüllungsteile (8, 9) aus Kunststoff gebildet sind.

13. Behälter (5) nach Anspruch 12, **dadurch gekennzeichnet, dass** die Behälterteile (6, 7) und Umhüllungsteile (8, 9) aus transparentem Kunststoff gebildet sind.

14. Behälter (5) nach Anspruch 12 oder 13, **dadurch gekennzeichnet, dass** der Kunststoff sterilisierbar, insbesondere gammmasterilisierbar, ist.

15. Behälter (5) nach einem der Ansprüche 12 bis 14, **dadurch gekennzeichnet, dass** die Behälterteile (6, 7) und Umhüllungsteile (8, 9) aus Polyolefinen, insbesondere Polypropylen oder Polyäthylen, gebildet sind.

16. Behälter (5) nach einem der Ansprüche 12 bis 15, **dadurch gekennzeichnet, dass** die Behälterteile (6, 7) und die Umhüllungsteile (8, 9) im Spritzgussverfahren herstellbar sind.

17. Behälter (5) nach einem der Ansprüche 1 bis 16, **dadurch gekennzeichnet, dass** die zumindest eine Entnahmeeinrichtung (11, 11') durch zumindest eine Durchstechmembran gebildet ist.

18. Behälter (5) nach einem der Ansprüche 1 bis 17, **dadurch gekennzeichnet, dass** die zumindest eine Entnahmeeinrichtung (11, 11') durch zumindest eine Nadel (12, 12') gebildet ist.

19. Behälter (5) nach Anspruch 18, **dadurch gekennzeichnet, dass** die zumindest eine Nadel (12, 12') aus Kunststoff gebildet ist.

20. Behälter (5) nach Anspruch 18, **dadurch gekennzeichnet, dass** die zumindest eine Nadel (12, 12') aus Metall gebildet ist.

21. Behälter (5) nach einem der Ansprüche 18 bis 20, **dadurch gekennzeichnet, dass** das zumindest eine Element mit zumindest einem Verbindungsstück (16, 16') verbunden ist.

22. Behälter (5) nach Anspruch 21, **dadurch gekennzeichnet, dass** das zumindest eine Verbindungsstück (16, 16') durch zumindest einen Schnellverschluss gebildet ist.

23. Behälter (5) nach einem der Ansprüche 1 bis 22, **dadurch gekennzeichnet, dass** eine sterile Verpackung (15) vorgesehen ist.

## Claims

1. A container (5) for receiving at least one non-sterile vessel (1, 1'), in particular a pierceable ampoule with a pierceable rubber stopper (3), containing a removable substance (2, 2'), and for introducing the at least one non-sterile vessel (1, 1') into a sterile region (I), said container comprising at least two interconnectable parts (6, 7) which, in their interconnected state, are designed to receive the at least one vessel (1, 1'), one container part (7) including at least one means (11, 11') for removing the respective substance (2, 2') from the at least one vessel (1, 1'), **characterised in that** said container parts (6, 7) are sterilised and surrounded by a removable envelope (10) which consists of at least two parts (8, 9), wherein the container parts (6, 7) are surrounded by respectively assigned envelope parts (8, 9) such that the container parts (6, 7) need not be touched when separating the container parts (6, 7) for opening the container (5), and wherein the envelope parts (8, 9) can be pulled off for removing the envelope (10).

2. The container (5) according to claim 1, **characterised in that** the envelope (10) is at least partially formed of an elastic material, in particular of an elastic synthetic material.

3. The container (5) according to claim 1 or 2, **characterised in that** a projection (13, 14) is provided on each envelope part (8, 9).

4. The container (5) according to any one of claims 1 to 3, **characterised in that** the envelope parts (8, 9) comprise ring elements (18, 19) which, in the closed state of the container (5), preferably contact each other.

5. The container (5) according to any one of claims 1 to 4, **characterised in that** the envelope parts (8, 9) comprise elements (20, 21) for a releasable connection with the container parts (6, 7), which are formed by complementarily shaped elements on the container parts (6, 7) and on the envelope parts (8, 9).

6. The container (5) according to claim 5, **characterised in that** a means (22) for releasing the connecting elements (20, 21), when the container (5) is completely closed, are provided, which are formed by complementarily shaped inclined end faces (23, 24) of the envelope parts (6, 7), and of their ring elements (18, 19), respectively, so that, when the container (5)is completely closed, the connecting elements (20, 21) of the envelope parts (6, 7) are releasable with the container parts (8, 9).

7. The container (5) according to any one of claims 1 to 6, **characterised in that** at least two, substantially equally sized container parts (6, 7) are provided.

8. The container (5) according to any one of claims 1 to 7, **characterised in that** the container parts (6, 7) are interconnectable via plug-in connections.

9. The container (5) according to any one of claims 1 to 7, **characterised in that** the container parts (6, 7) are interconnectable via a thread.

10. The container (5) according to any one of claims 1 to 9, **characterised in that** snap noses or the like are provided to prevent an undesired separation of the interconnected container parts (6, 7).

11. The container (5) according to any one of claims 1 to 10, **characterised in that** the container parts (6, 7) are cylindrically shaped.

12. The container (5) according to any one of claims 1 to 11, **characterised in that** the container parts (6, 7) and the envelope parts (8, 9) are made of a synthetic material.

13. The container (5) according to claim 12, **characterised in that** the container parts (6, 7) and the envelope parts (8, 9) are made of a transparent synthetic material.

14. The container (5) according to claim 12 or 13, **characterised in that** the synthetic material is capable of being sterilized, in particular sterilized by gamma radiation.

15. The container (5) according to any one of claims 12 to 14, **characterised in that** the container parts (6, 7) and the envelope parts (8, 9) are made of polyolefins, in particular polypropylene or polyethylene.

16. The container (5) according to any one of claims 12 to 15, **characterised in that** the container parts (6, 7) and the envelope parts (8, 9) can be produced by an injection moulding process.

17. The container (5) according to any one of claims 1 to 16, **characterised in that** the at least one removing means (11, 11') is formed by at least one pierceable membrane.

18. The container (5) according to any one of claims 1 to 17, **characterised in that** the at least one removing means (11, 11') is formed by at least one needle (12, 12').

19. The container (5) according to claim 18, **characterised in that** the at least one needle (12, 12') is made of a synthetic material.

20. The container (5) according to claim 18, **characterised in that** the at least one needle (12, 12') is made of metal.

21. The container (5) according to any one of claims 18 to 20, **characterised in that** the at least one element is connected to at least one connecting member (16, 16').

22. The container (5) according to claim 21, **characterised in that** the at least one connecting member (16, 16') is formed by at least one quick lock.

23. The container (5) according to any one of claims 1 to 22, **characterised in that** a sterile package (15) is provided.

## Revendications

1. Conteneur (5) pour le logement d'au moins un récipient non stérile (1, 1') contenant une substance prélevable (2, 2'), plus particulièrement une ampoule avec un bouchon en caoutchouc perforable (3) et pour l'insertion de l'au moins un récipient non stérile (1, 1') dans une zone stérile (I), avec au moins deux parties (6, 7) pouvant être reliées entre elles, qui sont conçues, lorsqu'elles sont reliées entre elles, pour le logement de l'au moins un récipient (1, 1'), une partie de conteneur (7) comprenant au moins un dispositif (11, 11') pour le prélèvement de la substance (2, 2') à partir de l'au moins un récipient (1, 1'), **caractérisé en ce que** les parties du conteneur (6, 7) sont stérilisées et sont entourées d'une enveloppe amovible (10) constituée d'au moins deux parties (8, 9), les parties du conteneur (6, 7) étant entourées chacune par des parties de l'enveloppe (8, 9) correspondantes, de façon à ce que les parties du conteneur (6, 7) ne doivent pas être touchées lors de la séparation des parties du conteneur (6, 7) pour l'ouverture du conteneur (5) et les parties de l'enveloppe (8, 9) pouvant être détachées pour retirer l'enveloppe (10).

2. Conteneur (5) selon la revendication 1, **caractérisé en ce que** l'enveloppe (10) est constituée au moins partiellement d'un matériau élastique, plus particulièrement d'une matière plastique élastique.

3. Conteneur (5) selon la revendication 1 ou 2, **caractérisé en ce que**, sur chaque partie de l'enveloppe (8, 9), est prévu un prolongement (13, 14).

4. Conteneur (5) selon l'une des revendications 1 à 3, **caractérisé en ce que** les parties de l'enveloppe (8, 9) comprennent des éléments annulaires (18, 19) qui se touchent de préférence mutuellement lorsque le conteneur (5) est fermé.

5. Conteneur (5) selon l'une des revendications 1 à 4, **caractérisé en ce que** les parties de l'enveloppe (8, 9) comprennent des éléments (20, 21) pour la liaison amovible avec les parties du conteneur (6, 7), qui sont constitués d'éléments complémentaires sur les parties du conteneur (6, 7) et les parties de l'enveloppe (8, 9).

6. Conteneur (5) selon la revendication 5, **caractérisé en ce qu'**un dispositif (22) est prévu pour le détachement des éléments de liaison (20, 21) lorsque le conteneur (5) est entièrement fermé, qui est constitué de surfaces d'extrémité (23, 24), obliques et complémentaires entre elles, des parties de l'enveloppe (6, 7) ou de leurs éléments annulaires (18, 19), de façon à ce que, lorsque le conteneur (5) est entièrement fermé, les éléments de liaison (20, 21) des parties de l'enveloppe (6, 7) puissent être détachées avec les parties du conteneur (8, 9).

7. Conteneur (5) selon l'une des revendications 1 à 6, **caractérisé en ce qu'**au moins deux parties de conteneur (6, 7) de tailles globalement identiques sont prévues.

8. Conteneur (5) selon l'une des revendications 1 à 7, **caractérisé en ce que** les parties du conteneur (6, 7) peuvent être reliées entre elles par l'intermédiaire d'une liaison par enfichage.

9. Conteneur (5) selon l'une des revendications 1 à 7, **caractérisé en ce que** les parties du conteneur (6, 7) peuvent être reliées entre elles par l'intermédiaire d'un filetage.

10. Conteneur (5) selon l'une des revendications 1 à 9, **caractérisé en ce que** des embouts d'encliquetage ou autre sont prévus pour empêcher une séparation non souhaitée des parties de conteneur (6, 7) reliées entre elles.

11. Conteneur (5) selon l'une des revendications 1 à 10, **caractérisé en ce que** les parties du conteneur (6, 7) présentent une forme cylindrique.

12. Conteneur (5) selon l'une des revendications 1 à 11, **caractérisé en ce que** les parties du conteneur (6, 7) et les parties de l'enveloppe (8, 9) sont constituées de matière plastique.

13. Conteneur (5) selon la revendication 12, **caractérisé en ce que** les parties du conteneur (6, 7) et les parties de l'enveloppe (8, 9) sont constituées d'une matière plastique transparente.

14. Conteneur (5) selon la revendication 12 ou 13, **caractérisé en ce que** la matière plastique est stérilisable, plus particulièrement stérilisable aux rayons gamma.

15. Conteneur (5) selon l'une des revendications 12 à 14, **caractérisé en ce que** les parties du conteneur (6, 7) et les parties de l'enveloppe (8, 9) sont constituées de polyoléfines, plus particulièrement de polypropylène ou de polyéthylène.

16. Conteneur (5) selon l'une des revendications 12 à 15, **caractérisé en ce que** les parties du conteneur (6, 7) et les parties de l'enveloppe (8, 9) peuvent être fabriqués à l'aide d'un procédé de moulage par injection.

17. Conteneur (5) selon l'une des revendications 1 à 16, **caractérisé en ce que** l'au moins un dispositif de prélèvement (11, 11') est constitué d'au moins une membrane perforable.

18. Conteneur (5) selon l'une des revendications 1 à 17, **caractérisé en ce que** l'au moins un dispositif de prélèvement (11, 11') est constitué d'au moins une aiguille (12, 12').

19. Conteneur (5) selon la revendication 18, **caractérisé en ce que** l'au moins une aiguille (12, 12') est constituée de matière plastique.

20. Conteneur (5) selon la revendication 18, **caractérisé en ce que** l'au moins une aiguille (12, 12') est constituée de métal.

21. Conteneur (5) selon l'une des revendications 18 à 20, **caractérisé en ce que** l'au moins un élément est relié avec au moins un élément de liaison (16, 16').

22. Conteneur (5) selon la revendication 21, **caractérisé en ce que** l'au moins un élément de liaison (16, 16') est constitué d'au moins une fermeture rapide.

23. Conteneur (5) selon l'une des revendications 1 à 22, **caractérisé en ce qu'**un emballage stérile (15) est prévu.
